# EUROPÄISCHE PATENTSCHRIFT

(11) **EP 3 729 072 B1**
(45) Veröffentlichungstag und Bekanntmachung des Hinweises auf die Patenterteilung: **16.03.2022**
(21) Anmeldenummer: 18826330.5
(22) Anmeldetag: 19.12.2018
(51) Int. Cl.: G01N 33/00

(54) **VORRICHTUNG ZUR UNTERSUCHUNG EINER ATMOSPHÄRE SOWIE VERWENDUNG DER VORRICHTUNG**
DEVICE FOR EXAMINING AN ATMOSPHERE AND USE OF THE DEVICE
DISPOSITIF POUR ANALYSER UNE ATMOSPHÈRE ET UTILISATION DU DISPOSITIF

(30) Priorität: 20.12.2017 DE 102017130755
(43) Veröffentlichungstag der Anmeldung: 28.10.2020
(73) Patentinhaber: Bilfinger Noell GmbH, 97080 Würzburg (DE)
(72) Erfinder: BOFFO, Cristian, 60510 Batavia, Illinois (US); PFEUFFER, Tatjana, 97078 Würzburg (DE)
(74) Vertreter: Cohausz & Florack
(86) Internationale Anmeldenummer: PCT/EP2018/085872
(87) Internationale Veröffentlichungsnummer: WO 2019/121936

(56) Entgegenhaltungen:
- DD-A5- 287 098
- JP-A- 2017 096 710
- BRIAN M. LERNER ET AL: "An Improved, Automated Whole-Air Sampler and Gas Chromatography Mass Spectrometry Analysis System for Volatile Organic Compounds in the Atmosphere", ATMOSPHERIC MEASUREMENT TECHNIQUES DISCUSSIONS, 11. Juli 2016 (2016-07-11), Seiten 1-40, XP055575927, DOI: 10.5194/amt-2016-210
- R.G. Ross: "Aerospace Coolers: A 50-Year Quest for Long-Life Cryogenic Cooling in Space" In: "Cryogenic Engineering", 1. Januar 2007 (2007-01-01), Springer, US, XP055576383, ISBN: 978-0-387-33324-3 Seiten 225-284, DOI: 10.1007/0-387-46896-X_11, Seiten 22-30
- Ronald G. Ross: "Refrigeration Systems for Achieving Cryogenic Temperatures", Chapter 6 of Low Temperature Materials and Mechanisms (Ed. by Yoseph Bar-Cohen), 1. Januar 2016 (2016-01-01), XP055576376, Gefunden im Internet: URL:https://www2.jpl.nasa.gov/adv_tech/coo lers/Cool_ppr/Chap%206-Refrig%20Sys%20for% 20Achiev%20Cryo%20Temps_2016.pdf [gefunden am 2019-04-02]

## Beschreibung

Die Erfindung betrifft eine Vorrichtung zur Untersuchung einer Atmosphäre sowie eine Verwendung der Vorrichtung.

Untersuchungen der Zusammensetzung der Atmosphäre sowie der Wechselwirkung ihrer Bestandteile (z.B. Aerosole und Verunreinigungen) untereinander sind ein wichtiger Teil der aktuellen Klimaforschung.

Es existieren Vorrichtungen zur Untersuchungen der Atmosphäre mit einer Atmosphärenanalysekammer. Es hat sich herausgestellt, dass eine zuverlässige, effektive und möglichst wartungsarme Kühlung der Atmosphärenanalysekammer wichtig für den Einsatz derartiger Vorrichtungen sind. Vorrichtungen mit Kühlkreisläufen, in denen eine Kühlflüssigkeit durch Leitungen, Wärmetauscher und Kühlaggregat zirkuliert, haben sich als recht aufwändig und wartungsintensiv erwiesen und sind daher für den Feldeinsatz schlecht geeignet. Dasselbe gilt für Systeme, die ein Temperiergas benutzen.

Hiervon ausgehend liegt der vorliegenden Erfindung die Aufgabe zu Grunde, eine Vorrichtung zur Untersuchung der Atmosphäre bereitzustellen, die zuverlässiger, effektiver und möglichst wartungsärmer funktioniert.

Diese Aufgabe wird erfindungsgemäß gelöst durch eine Vorrichtung zur Untersuchung einer Atmosphäre mit einer Atmosphärenanalysekammer und mit einem Kryokühler, der zur Kühlung der Atmosphärenanalysekammer thermisch mit der Atmosphärenanalysekammer gekoppelt ist, wobei ein optischer Partikelzähler an der Atmosphärenanalysekammer angebracht ist. Der Kryokühler kann insbesondere mit einem Bereich der die Atmosphärenanalysekammer bildenden Wand gekoppelt sein.

Durch die Verwendung eines Kryokühlers zur Kühlung der Atmosphärenanalysekammer anstelle eines Kühlkreislaufs wird eine platzsparende und wartungsarme Kühlung der Atmosphärenanalysekammer erreicht. Mit einem Kryokühler sind weiterhin eine hohe Kühlleistung und eine gute Temperaturkontrolle möglich. Zudem erlaubt ein Kryokühler einen deutlich erweiterten Betriebstemperaturbereich, sogar bis unter - 200°C.

Bei der Atmosphäre kann es sich um eine natürliche Atmosphäre handeln, insbesondere um die natürliche Erdatmosphäre, oder auch um eine künstliche Atmosphäre.

Bei dem Kryokühler kann es sich beispielsweise um einen Gifford-McMahon-Kryokühler, einen Pulsrohr-Kryokühler oder um einen Stirling-Kryokühler handeln. Der Kryokühler kann ein oder mehrstufig ausgebildet sein, vorzugsweise jedoch einstufig.

Die Aufgabe wird weiterhin gelöst durch die Verwendung der zuvor beschriebenen Vorrichtung zur Untersuchung einer Atmosphäre, insbesondere der Zusammensetzung einer Atmosphäre.

Im Folgenden werden verschiedene Ausführungsformen beschrieben, die sowohl für die Vorrichtung als auch für die Verwendung gelten. Weiterhin können die Ausführungsformen auch miteinander kombiniert werden.

Bei einer Ausführungsform weist die Atmosphärenanalysekammer einen Einlass auf. Auf diese Weise kann die zu untersuchende Atmosphäre in die Atmosphärenanalysekammer geleitet werden.

Bei einer weiteren Ausführungsform weist die Atmosphärenanalysekammer zwei, insbesondere auf gegenüberliegenden Seiten der Atmosphärenanalysekammer angeordnete Einlässe auf. Auf diese Weise kann die zu untersuchende Atmosphäre durch die Atmosphärenanalysekammer hindurch geleitet werden.

Bei einer Ausführungsform weist der Kryokühler einen thermisch mit der Atmosphärenanalysekammer gekoppelten Kaltkopf auf. Der Kryokühler kann auch mehrere thermisch mit der Atmosphärenanalysekammer gekoppelte Kaltköpfe aufweisen. Weiterhin können auch mehrere Kryokühler vorgesehen sein, die bzw. deren Kaltköpfe thermisch mit der Atmosphärenanalysekammer gekoppelt sind.

Bei einer Ausführungsform weist die Vorrichtung eine die Atmosphärenanalysekammer umgebende, vorzugsweise geschlossene Außenkammer auf. Die Außenkammer kann beispielsweise ringartig um die Atmosphärenanalysekammer angeordnet sein. Durch das Vorsehen einer solchen Außenkammer kann die Atmosphärenanalysekammer zumindest bereichsweise thermisch von der Umgebung isoliert werden.

Bei einer Ausführungsform steht die Außenkammer unter Unterdruck, insbesondere Vakuum. Dadurch wird die thermische Isolationswirkung der Außenkammer verbessert.

Bei einer Ausführungsform werden die Atmosphärenanalysekammer und die Außenkammer durch einen inneren Behälter und einen diesen umgebenden äußeren Behälter gebildet. Die Wand des inneren Behälters bildet vorzugsweise die Atmosphärenanalysekammer bzw. deren Wand. Die Wand des inneren Behälters und die Wand des äußeren Behälters bilden zusammen vorzugsweise die Außenkammer bzw. deren Wand.

Bei einer Ausführungsform ist der Kaltkopf des Kryokühlers innerhalb der Außenkammer angeordnet. Auf diese Weise wird auch der Kaltkopf des Kryokühlers thermisch gegenüber der Umgebung isoliert, wodurch die Kühleffizienz verbessert wird.

Bei einer Ausführungsform ist der Kaltkopf des Kryokühlers mittels einer oder mehrerer thermisch leitenden Verbindungen mit der Atmosphärenanalysekammer thermisch gekoppelt. Die eine oder mehreren thermisch leitenden Verbindungen sind vorzugsweise innerhalb der Außenkammer angeordnet. Auf diese Weise wird die Kühleffizienz weiter verbessert.

Zur effektiveren und gleichmäßigeren Kühlung der Atmosphärenanalysekammer können insbesondere mehrere thermisch leitende Verbindungen zwischen dem Kaltkopf und verschiedenen Stellen der Atmosphärenkammer, insbesondere verschiedenen Stellen der die Atmosphärenkammer bildenden Wand vorgesehen sein.

Als thermisch leitende Verbindungen sind insbesondere flexible Verbindungen und/oder Verbindungen aus einem thermisch gut leitfähigen Material wie Kupfer oder Aluminium geeignet. Beispielsweise können die eine oder mehreren Verbindungen durch eine bzw. mehrere Kupfer- oder Aluminiumlitzen gebildet werden.

Bei einer Ausführungsform ist die Wand der Atmosphärenanalysekammer und/oder der Außenkammer aus Edelstahl, Aluminium oder Kupfer gebildet.

Bei einer Ausführungsform weist die Wand der Atmosphärenanalysekammer auf der Innenseite und/oder auf der Außenseite eine Beschichtung auf. Auf diese Weise kann eine bessere und gleichmäßigere Verteilung der Temperatur an der Atmosphärenanalysekammer erreicht werden. Zu diesem Zweck besteht die Beschichtung vorzugsweise aus einem Material mit guter thermischer Leitfähigkeit, beispielsweise aus Kupfer.

Bei einer Ausführungsform sind ein oder mehrere Heizelemente an der Atmosphärenanalysekammer angebracht oder thermisch mit dieser gekoppelt. Auf diese Weise kann die Temperatur der Atmosphärenanalysekammer besser geregelt werden. Die ein oder mehreren Heizelemente können beispielsweise mit einem oder verschiedenen Wandbereichen der Atmosphärenkammer gekoppelt oder daran angebracht sein. Vorzugsweise werden ein oder mehrere resistive Heizelemente verwendet.

Bei einer Ausführungsform sind ein oder mehrere Heizelemente an dem Kaltkopf und/oder an einer oder mehreren thermisch leitenden Verbindungen des Kaltkopfs mit einem oder mehreren Wandbereichen der Atmosphärenanalysekammer angebracht oder thermisch damit gekoppelt. Auf diese Weise kann eine zusätzliche Wärmelast für den Kaltkopf bzw. die Verbindungen aktiviert werden, wodurch der Temperatursteuerungsprozess unterstützt werden kann. Die ein oder mehreren Heizelemente sind vorzugsweise resistiv.

Bei einer Ausführungsform ist innerhalb der Außenkammer ein Schild vorgesehen, um Wärmestrahlung zwischen einem Wandbereich der Außenkammer und einem Wandbereich der Atmosphärenanalysekammer zu reduzieren. Auf diese Weise kann die Wärmelast durch Strahlung auf die Wand der Atmosphärenanalysekammer kontrolliert und reduziert werden. Das Schild ist vorzugsweise derart beschaffen, dass es von der Außenseite der Außenkammer kommende Wärmestrahlung zumindest teilweise reflektiert. Zu diesem Zweck kann der Schild insbesondere eine Mehrschichtisolierung aufweisen.

Bei einer Ausführungsform ist der Schild zu dessen Kühlung thermisch mit einem Kryokühler gekoppelt. Bei dem Kryokühler kann es sich um einen Kryokühler zur Kühlung der Atmosphärenanalysekammer handeln oder auch um einen separaten Kryokühler. Der mit dem Schild gekoppelte Kryokühler ist vorzugsweise dazu eingerichtet, den Schild auf einen vordefinierten Temperaturbereich zu kühlen. Auf diese Weise kann die Wärmestrahlung besser von der Atmosphärenanalysekammer abgeschirmt werden.

Bei einer weiteren Ausführungsform kann direkt auf der Außenwand der Atmosphärenanalysekammer reflektierende Mehrschichtisolation aufgebracht werden, um den Eintrag von Strahlungswärme zu reduzieren.

Bei einer Ausführungsform ist ein Sensor oder Analysegerät zur Analyse der Atmosphäre in der Atmosphärenanalysekammer angeordnet oder daran angeschlossen. Auf diese Weise kann die in die Atmosphärenanalysekammer gelangende Atmosphäre analysiert werden, insbesondere hinsichtlich deren Zusammensetzung. Weitere Messgeräte können auch an oder in den Zufluss der Atmosphärenanalysekammer oder deren Abfluss angeordnet sein.

Die verwendeten Messinstrumente sind vorzugsweise geeignet, Druckverhältnisse und/oder Temperaturen in der Atmosphärenanalysekammer zu messen und aufzuzeichnen. Erfindungsgemäß ist ein optischer Partikelzähler an der Atmosphärenanalysekammer angebracht. Weiterhin können Messgeräte wie Massenspektrometer für kleine Teilchen an der Atmosphärenanalysekammer angebracht sein. Weitere vorteilhafte Messgeräte umfassen Gasanalysegeräte und interferometrische Spektrometer aus verschiedenen Wellenlängenbereichen.

Weitere Merkmale und Vorteile der Vorrichtung und der Verwendung ergeben sich aus der nachfolgenden Beschreibung von Ausführungsbeispielen, wobei auf die beigefügte Zeichnung Bezug genommen wird.

In der Zeichnung zeigen
- Fig. 1: eine erste Vorrichtung mit einer Kühlkreislauf-Kühlung,
- Fig. 2: eine zweite Vorrichtung mit einer Kühlkreislauf-Kühlung,
- Fig. 3: ein erstes Ausführungsbeispiel der Vorrichtung mit einem Kryokühler,
- Fig. 4: ein zweites Ausführungsbeispiel der Vorrichtung mit einem Kryokühler.

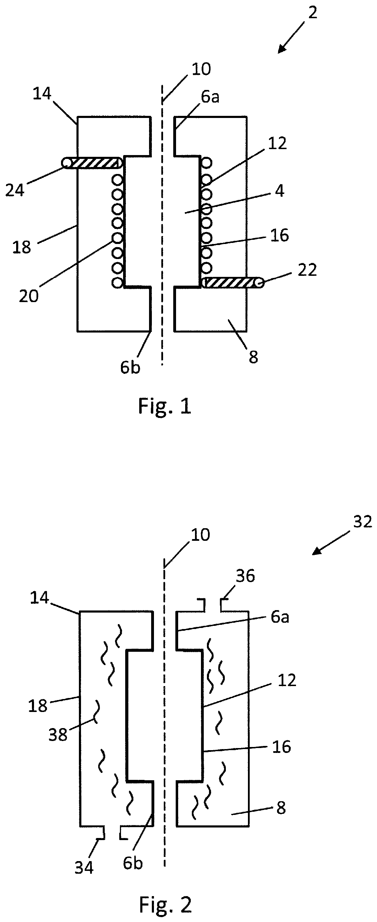

Fig. 1 zeigt eine Vorrichtung zur Untersuchung der Atmosphäre mit einer Atmosphärenanalysekammer mit einer Kühlkreislauf-Kühlung in schematischer Schnittansicht.

Die Vorrichtung 2 umfasst eine Atmosphärenanalysekammer 4 mit zwei gegenüberliegenden Einlässen 6a-b und eine diese umgebende geschlossene Außenkammer 8. Die Atmosphärenanalysekammer 4 und die Außenkammer 8 sind vorliegend rotationssymmetrisch zu der Achse 10, die durch die beiden Einlässe 6a-b verläuft.

Die Atmosphärenanalysekammer 4 und die Außenkammer 8 werden bei der Vorrichtung durch einen inneren Behälter 12 und einen diesen umgebenden äußeren Behälter 14 gebildet. Die Wand 16 des inneren Behälters 12 bildet die Wand der Atmosphärenanalysekammer 4. Die Wand 16 bildet zudem mit der Wand 18 des äußeren Behälters 14 die Wand der Außenkammer 8.

Zur Kühlung der Atmosphärenanalysekammer 4 ist auf dem inneren Behälter 12 ein Rohrwändel 20 aufgebracht, durch den im Betrieb eine Kühlflüssigkeit fließt. Zu diesem Zweck sind ein Kühlflüssigkeitsvorlauf 22 und ein Kühlflüssigkeitsrücklauf 24 vorgesehen, über die das Rohrwändel 20 in einen Kühlkreislauf eingebettet ist.

Fig. 2 zeigt eine zweite Vorrichtung zur Untersuchung der Atmosphäre mit einer Atmosphärenanalysekammer mit einer Kühlkreislauf-Kühlung in schematischer Schnittansicht.

Die Vorrichtung 32 weist einen ähnlichen Aufbau auf wie die Vorrichtung 2. Einander entsprechende Komponenten sind mit gleichen Bezugszeichen versehen.

Die Vorrichtung 32 unterscheidet sich dadurch von der Vorrichtung 2, dass zur Kühlung der Atmosphärenanalysekammer 4 anstelle des kühlflüssigkeitdurchströmbaren Rohrwändels 20 eine Kühlung durch ein Temperiergas aus einem Kühlkreislauf vorgesehen ist.

Zu diesem Zweck sind am äußeren Behälter ein Einlass 34 und ein Auslass 36 für ein Temperiergas eines Kühlkreislaufs vorgesehen. Im Betrieb wird ein gekühltes Temperiergas 38 durch den Einlass 34 in die Außenkammer 8 geleitet, das nach Durchströmen der Außenkammer 8 durch den Auslass 36 wieder aus der Außenkammer 8 heraus gelangt. Das Temperiergas kühlt die Wand 16 des inneren Behälters 12 und dadurch die Atmosphärenanalysekammer 4.

Die Vorrichtungen 2 und 32 erlaubt eine Kühlung der Atmosphärenanalysekammer 4. Jedoch hat sich die Kühlung mittels eines Kühlkreislaufs mittels Kühlflüssigkeit wie bei Vorrichtung 2 oder mittels Temperiergas wie bei Vorrichtung 32 als recht aufwändig und wartungsintensiv erwiesen.

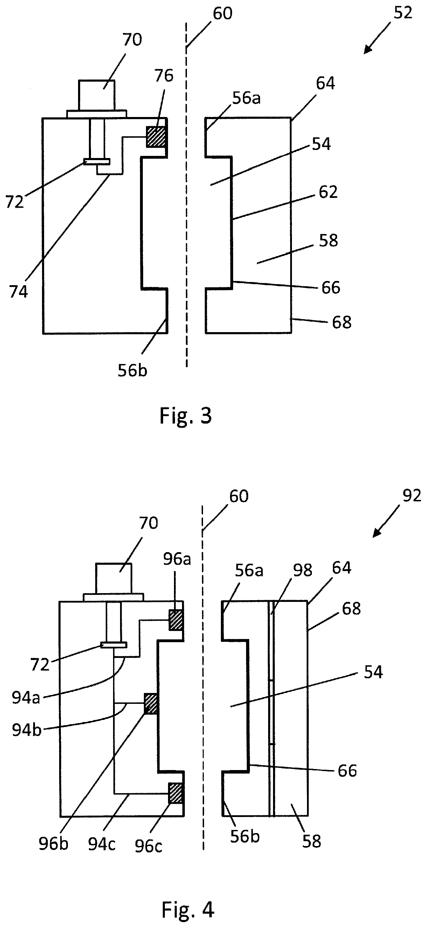

Fig. 3 zeigt nun ein erstes Ausführungsbeispiel der Vorrichtung mit einem Kryokühler in schematischer Schnittansicht.

Die Vorrichtung 52 umfasst eine Atmosphärenanalysekammer 54 mit zwei gegenüberliegenden Einlässen 56a-b und eine diese umgebende geschlossene Außenkammer 58, die unter Unterdruck, vorzugsweise unter Vakuum, steht. Die Atmosphärenanalysekammer 54 und die Außenkammer 58 sind vorliegend rotationssymmetrisch zu der Achse 60, die durch die beiden Einlässe 56a-b verläuft.

Die Atmosphärenanalysekammer 54 und die Außenkammer 8 werden bei der Vorrichtung 52 durch einen inneren Behälter 62 und einen diesen umgebenden äußeren Behälter 64 gebildet. Die Wand 66 des inneren Behälters 62 bildet die Wand der Atmosphärenanalysekammer 54. Die Wand 66 bildet zudem mit der Wand 68 des äußeren Behälters 64 die Wand der Außenkammer 58.

Zur Kühlung der Atmosphärenanalysekammer 54 ist ein Kryokühler 70 vorgesehen. Bei dem Kryokühler 70 kann es sich zum Beispiel um einen Gifford-McMahon-Kryokühler, einen Pulsrohr-Kryokühler oder um einen Stirling-Kryokühler handeln. Der Kryokühler ist vorzugsweise einstufig. Der Kryokühler weist einen Kühlkopf 72 auf, der in der Außenkammer 58 angeordnet ist. Der Kühlkopf 72 ist über eine thermisch leitende Verbindung 74 mittels einer thermischen Anbindung 76 thermisch mit der Wand 66 und dadurch mit der Atmosphärenanalysekammer 54 gekoppelt. Bei der thermisch leitenden Verbindung 74 kann es sich beispielsweise um eine Kupferlitze oder ein Kupferlitzengeflecht handeln. Bei der thermischen Anbindung 76 kann es sich beispielsweise um eine Schraubverbindung, eine Lötverbindung oder um eine Klemmverbindung handeln. In Fig. 3 ist der Kühlkopf 72 im Bereich des Einlasses 56a mit der Wand 66 verbunden. Die Wand 66 ist auf der Innenseite (d.h. auf der in der Atmosphärenanalysekammer 54 liegenden Seite) und/oder auf der Außenseite (d.h. auf der in der Außenkammer 58 liegenden Seite) mit einer Kupferbeschichtung versehen, wodurch eine gleichmäßigere Temperierung der Atmosphärenanalysekammer 54 erreicht wird.

Fig. 4 zeigt ein zweites Ausführungsbeispiel der Vorrichtung mit einem Kryokühler in schematischer Schnittansicht

Die Vorrichtung 92 weist einen ähnlichen Aufbau auf wie die Vorrichtung 52. Einander entsprechende Komponenten sind mit gleichen Bezugszeichen versehen.

Die Vorrichtung 92 unterscheidet sich dadurch von der Vorrichtung 52, dass der Kühlkopf 72 über mehrere thermisch leitende Verbindungen 94a-c mittels jeweiliger thermischen Anbindungen 96a-c thermisch mit der Wand 66 und dadurch mit der Atmosphärenanalysekammer 54 gekoppelt ist. In Fig. 4 ist der Kühlkopf 72 im Bereich des Einlasses 56a, im Bereich des Einlasses 56b sowie in einem dazwischen liegenden Bereich mit der Wand 66 verbunden.

Innerhalb der Außenkammer 58 ist zwischen der Wand 66 des inneren Behälters 62 und der Wand 68 des äußeren Behälters 64 ein Schild 98 angeordnet, dass die Übertragung von Wärmestrahlung von der Wand 68 zur Wand 66 reduziert. Für eine noch effektivere Reduzierung der Wärmestrahlungsübertragung kann der Schild 98 thermisch mit dem Kühlkopf 72 verbunden sein.

An dem Kühlkopf 72, an den thermisch leitenden Verbindungen 94a-c und/oder an der Wand 66 können ein oder mehrere ansteuerbare Heizelemente vorgesehen sein, die eine bessere Steuerung und/oder Regelung der Temperatur der Atmosphärenanalysekammer 54 ermöglichen.

Es hat sich herausgestellt, dass durch die Verwendung eines Kryokühlers 70 eine wartungsarme und effiziente Kühlung der Atmosphärenanalysekammer 54 ermöglicht wird. Eine besonders effiziente Kühlung kann mit den Ausgestaltungen gemäß der Vorrichtungen 52 und 92 erreicht werden.

## Patentansprüche

1. Vorrichtung (52, 92) zur Untersuchung einer Atmosphäre
- mit einer Atmosphärenanalysekammer (54) und
- mit einem Kryokühler (70), der zur Kühlung der Atmosphärenanalysekammer (54) thermisch mit der Atmosphärenanalysekammer (54) gekoppelt ist,
**dadurch gekennzeichnet,**
**dass** ein optischer Partikelzähler an der Atmosphärenanalysekammer (54) angebracht ist.

2. Vorrichtung nach Anspruch 1,
**dadurch gekennzeichnet, dass** die Atmosphärenanalysekammer (54) zwei, insbesondere auf gegenüberliegenden Seiten der Atmosphärenanalysekammer (54) angeordnete Einlässe (56a-b) aufweist.

3. Vorrichtung nach Anspruch 1 oder 2,
**dadurch gekennzeichnet, dass** der Kryokühler (70) einen thermisch mit der Atmosphärenanalysekammer (54) gekoppelten Kaltkopf (72) aufweist.

4. Vorrichtung nach einem der Ansprüche 1 bis 3,
**dadurch gekennzeichnet, dass** die Vorrichtung (52, 92) eine die Atmosphärenanalysekammer (54) umgebende, vorzugsweise geschlossene Außenkammer (58) aufweist.

5. Vorrichtung nach Anspruch 4,
**dadurch gekennzeichnet, dass** die Außenkammer (58) unter Unterdruck steht.

6. Vorrichtung nach Anspruch 4 oder 5,
**dadurch gekennzeichnet, dass** der Kaltkopf (72) des Kryokühlers (70) innerhalb der Außenkammer (58) angeordnet ist.

7. Vorrichtung nach einem der Ansprüche 3 bis 6,
**dadurch gekennzeichnet, dass** der Kaltkopf (72) des Kryokühlers (70) mittels einer oder mehrerer thermisch leitenden Verbindungen (74; 96a-c) mit der Atmosphärenanalysekammer (54) thermisch gekoppelt ist, wobei die eine oder mehreren thermisch leitenden Verbindungen (74; 96a-c) vorzugsweise innerhalb der Außenkammer (58) angeordnet sind.

8. Vorrichtung nach einem der Ansprüche 1 bis 7,
**dadurch gekennzeichnet, dass** die Wand (66) der Atmosphärenanalysekammer (54) und/oder die Wand (66, 68) der Außenkammer (58) aus Edelstahl, Aluminium oder Kupfer gebildet ist.

9. Vorrichtung nach einem der Ansprüche 1 bis 8,
**dadurch gekennzeichnet, dass** die Wand (66) der Atmosphärenanalysekammer (54) auf der Innenseite und/oder auf der Außenseite eine Beschichtung aufweist.

10. Vorrichtung nach einem der Ansprüche 1 bis 9,
**dadurch gekennzeichnet, dass** ein oder mehrere Heizelemente an der Atmosphärenanalysekammer (54) angebracht oder thermisch mit dieser gekoppelt sind.

11. Vorrichtung nach einem der Ansprüche 3 bis 10,
**dadurch gekennzeichnet, dass** ein oder mehrere Heizelemente an dem Kaltkopf (72) angebracht oder thermisch mit diesem gekoppelt sind.

12. Vorrichtung nach Anspruch einem der Ansprüche 4 bis 11,
**dadurch gekennzeichnet, dass** innerhalb der Außenkammer (54) ein Schild (98) vorgesehen ist, um Wärmestrahlung zwischen einem Wandbereich (68) der Außenkammer (58) und einem Wandbereich (66) der Atmosphärenanalysekammer (54) zu reduzieren.

13. Vorrichtung nach Anspruch 12,
**dadurch gekennzeichnet, dass** der Schild (98) zu dessen Kühlung thermisch mit einem Kryokühler (70) gekoppelt ist.

14. Vorrichtung nach einem der Ansprüche 1 bis 13,
**dadurch gekennzeichnet, dass** ein Sensor oder Analysegerät zur Analyse der Atmosphäre in der Atmosphärenanalysekammer (54) angeordnet oder daran angeschlossen ist.

15. Verwendung einer Vorrichtung (52, 92) nach einem der Ansprüche 1 bis 14 zur Untersuchung einer Atmosphäre, insbesondere der Zusammensetzung einer Atmosphäre.

## Claims

1. Device (52, 92) for examining an atmosphere,
- comprising an atmosphere analysis chamber (54) and
- a cryocooler (70) which is thermally coupled to the atmosphere analysis chamber (54) for cooling the atmosphere analysis chamber (54)
**characterized in**
**that** an optical particle counter is attached to the atmosphere analysis chamber.

2. Device according to claim 1,
**characterised in that** the atmosphere analysis chamber (54) has two inlets (56a-b), in particular arranged on opposite sides of the atmosphere analysis chamber (54).

3. Device according to claim 1 or 2,
**characterised in that** the cryocooler (70) has a cooling head (72) which is thermally coupled to the atmosphere analysis chamber (54).

4. Device according to any one of claims 1 to 3,
**characterised in that** the device (52, 92) has a preferably closed outer chamber (58) surrounding the atmosphere analysis chamber (54).

5. Device according to claim 4,
**characterised in that** the outer chamber (58) is under negative pressure.

6. Device according to claim 4 or 5,
**characterised in that** the cooling head (72) of the cryocooler (70) is arranged inside the outer chamber (58).

7. Device according to any one of claims 3 to 6,
**characterised in that** the cooling head (72) of the cryocooler (70) is thermally coupled to the atmosphere analysis chamber (54) by means of one or more thermally conductive connections (74; 96a-c), wherein the one or more thermally conductive connections (74; 96a-c) are preferably arranged within the outer chamber (58).

8. Device according to any one of claims 1 to 7,
**characterised in that** the wall (66) of the atmosphere analysis chamber (54) and/or the wall (66, 68) of the outer chamber (58) is made of stainless steel, aluminium or copper.

9. Device according to any one of claims 1 to 8,
**characterised in that** the wall (66) of the atmosphere analysis chamber (54) has a coating on the inner side and/or on the outer side.

10. Device according to any one of claims 1 to 9,
**characterised in that** one or more heating elements are attached or thermally coupled to the atmosphere analysis chamber (54).

11. Device according to any one of claims 3 to 10,
**characterised in that** one or more heating elements are attached or thermally coupled to the cooling head (72).

12. Device according to any one of claims 4 to 11,
**characterised in that** a shield (98) is provided inside the outer chamber (54) to reduce thermal radiation between a wall region (68) of the outer chamber (58) and a wall region (66) of the atmosphere analysis chamber (54).

13. Device according to claim 12,
**characterised in that** the shield (98) is thermally coupled to a cryocooler (70) for its cooling.

14. Device according to any one of claims 1 to 13,
**characterised in that** a sensor or analytical apparatus for analysing the atmosphere is arranged in or connected to the atmosphere analysis chamber (54).

15. Use of a device (52, 92) according to any one of claims 1 to 14 for examining an atmosphere, in particular the composition of an atmosphere.

## Revendications

1. Dispositif (52, 92) d'analyse d'une atmosphère
- avec une chambre d'analyse atmosphérique (54) et
- avec un refroidisseur cryogénique (70) qui est couplé thermiquement à la chambre d'analyse atmosphérique (54) pour refroidir la chambre d'analyse atmosphérique (54),
**caractérisé**
**en ce qu'**un compteur optique de particules est monté au niveau de la chambre d'analyse atmosphérique (54).

2. Dispositif selon la revendication 1,
**caractérisé en ce que** la chambre d'analyse atmosphérique (54) comporte deux entrées (56a-b), en particulier disposées sur des côtés opposés de la chambre d'analyse atmosphérique (54).

3. Dispositif selon la revendication 1 ou 2,
**caractérisé en ce que** le refroidisseur cryogénique (70) comporte une tête froide (72) couplée thermiquement à la chambre d'analyse atmosphérique (54).

4. Dispositif selon l'une des revendications 1 à 3,
**caractérisé en ce que** le dispositif (52, 92) comporte une chambre extérieure (58), de préférence fermée, entourant la chambre d'analyse atmosphérique (54).

5. Dispositif selon la revendication 4,
**caractérisé en ce que** la chambre extérieure (58) est sous pression négative.

6. Dispositif selon la revendication 4 ou 5,
**caractérisé en ce que** la tête froide (72) du refroidisseur cryogénique (70) est disposée à l'intérieur de la chambre extérieure (58).

7. Dispositif selon l'une des revendications 3 à 6,
**caractérisé en ce que** la tête froide (72) du refroidisseur cryogénique (70) est couplée thermiquement à la chambre d'analyse atmosphérique (54) au moyen d'une ou plusieurs connections (74 ; 96a-c) thermiquement conductrices, où l'une ou plusieurs connections (74 ; 96a-c) thermiquement conductrices sont de préférence disposées à l'intérieur de la chambre extérieure (58).

8. Dispositif selon l'une quelconque des revendications 1 à 7,
**caractérisé en ce que** la paroi (66) de la chambre d'analyse atmosphérique (54) et/ou la paroi (66, 68) de la chambre extérieure (58) sont formées en acier inoxydable, en aluminium ou en cuivre.

9. Dispositif selon l'une quelconque des revendications 1 à 8,
**caractérisé en ce que** la paroi (66) de la chambre d'analyse atmosphérique (54) comporte un revêtement sur la face interne et/ou sur la face externe.

10. Dispositif selon l'une quelconque des revendications 1 à 9,
**caractérisé en ce qu'**un ou plusieurs éléments chauffants sont montés au niveau de la chambre d'analyse atmosphérique (54) ou couplés thermiquement à celle-ci.

11. Dispositif selon l'une des revendications 3 à 10,
**caractérisé en ce qu'**un ou plusieurs éléments chauffants sont montés au niveau de la tête froide (72) ou couplés thermiquement à celle-ci.

12. Dispositif selon l'une quelconque des revendications 4 à 11,
**caractérisé en ce qu'**un écran (98) est prévu à l'intérieur de la chambre extérieure (54) pour réduire le rayonnement thermique entre une zone de paroi (68) de la chambre extérieure (58) et une zone de paroi (66) de la chambre d'analyse atmosphérique (54).

13. Dispositif selon la revendication 12,
**caractérisé en ce que** l'écran (98) est couplé thermiquement à un refroidisseur cryogénique (70) pour son refroidissement.

14. Dispositif selon l'une des revendications 1 à 13,
**caractérisé en ce qu'**un capteur ou un analyseur pour l'analyse de l'atmosphère est disposé dans la chambre d'analyse de l'atmosphère (54) ou est connecté à celle-ci.

15. Utilisation d'un dispositif (52, 92) selon l'une des revendications 1 à 14 pour analyser une atmosphère, en particulier la composition d'une atmosphère.
